# EUROPEAN PATENT SPECIFICATION

(11) **EP 1 245 235 B1**
(45) Date of publication and mention of the grant of the patent: **29.09.2004**
(21) Application number: 01128539.2
(22) Date of filing: 29.11.2001
(51) Int. Cl.: A61K 35/84, A61P 35/00, A61P 31/12, A61P 31/18

(54) **Method for extracting oleaginous substances from ganoderma lucidum spores**
Methode zur Extraktion von öligen Substanzen aus Ganoderma lucidum Sporen
Méthode d'extraction des substances oléagineuses des spores de Ganoderma lucidum

(30) Priority: 19.03.2001 US 810234; 19.03.2001 US 810213
(43) Date of publication of application: 02.10.2002
(73) Proprietor: Liu, Xin, Guangzhou (CN); Huang, Xiao-Ni, Guangzhou (CN); Chung, Peter Chee-Keung, Mongkok, Kowloon, Hong Kong (CN)
(72) Inventor: Liu, Xin, Guangzhou (CN); Huang, Xiao-Ni, Guangzhou (CN); Chung, Peter Chee-Keung, Mongkok, Kowloon, Hong Kong (CN)
(74) Representative: Prüfer, Lutz H., Dipl.-Phys.

(56) References cited:
- EP-A- 1 092 765
- DATABASE WPI Section Ch, Week 200062 Derwent Publications Ltd., London, GB; Class B04, AN 2000-639170 XP002208630 & CN 1 262 957 A (NANJING GEOGRAPHY & LAKE INST), 16 August 2000 (2000-08-16)
- MIN B S ET AL: "Triterpenes from the spores of Ganoderma lucidum and their cytotoxicity against meth-A and LLC tumor cells." CHEMICAL & PHARMACEUTICAL BULLETIN. JAPAN JUL 2000, vol. 48, no. 7, July 2000 (2000-07), pages 1026-1033, XP001098589 ISSN: 0009-2363
- MIN B S ET AL: "Triterpenes from the spores of Ganoderma lucidum and their inhibitory activity against HIV-1 protease." CHEMICAL & PHARMACEUTICAL BULLETIN. JAPAN OCT 1998, vol. 46, no. 10, October 1998 (1998-10), pages 1607-1612, XP002209094 ISSN: 0009-2363
- DATABASE WPI Section Ch, Week 200156 Derwent Publications Ltd., London, GB; Class B04, AN 2001-503343 XP002208631 & CN 1 298 741 A (UNIV ZHONGSHAN), 13 June 2001 (2001-06-13)
- DATABASE WPI Section Ch, Week 200146 Derwent Publications Ltd., London, GB; Class B04, AN 2001-426151 XP002208632 & CN 1 290 554 A (UNIV ZHONGSHAN), 11 April 2001 (2001-04-11)
- GUPTA S K ET AL: "Ganoderma lucidum: Partial characterization of spore and whole body antigenic extracts." JOURNAL OF INVESTIGATIONAL ALLERGOLOGY & CLINICAL IMMUNOLOGY, vol. 10, no. 2, March 2000 (2000-03), pages 83-89, XP002209095 ISSN: 1018-9068

## Description

### FIELD OF THE INVENTION

The present invention relates to a method for extracting oleaginous substances from *Ganoderma lucidum* spores using supercritical fluid carbon dioxide (SCF-CO₂). The spores are germination-activated and epispore-broken before SCF-CO₂ extraction.

### BACKGROUND OF THE INVENTION

*Ganoderma* (*Ganoderma lucidum Leyss* ex Fr. Karst) is a polyporous fungus. It belongs to the class of Basidiomycetes, the family of Polypolaceae, and the genus of *Ganoderma.* In Chinese folklore, *Ganoderma* has been regarded as a panacea, which is probably due to certain efficacy of *Ganoderma* in treating many diseases. Some of the known medicinal or therapeutic effects of *Ganoderma* include treating patients with chronic bronchitis, chronic viral hepatitis, coronary heart disease, granulocytopenia, chronic Keshan disease, neurasthenia, progressive muscular dystrophy, atrophic myotonia and certain neurological diseases (See e.g., Liu et al., Chinese Medical Journal, 92:496-500 (1979)). There are also reports on *Ganoderma* as anti-HIV agent (See e.g., El-Mekkawy et al., Phytochemistry, 49: 1651-1657 (1998); Min et al., Chem. Pharm. Bull, 46: 1607-1612 (1998)), or for having anti-tumor, cardiovascular, antiviral, antibacterial, antiparasitic, and immune modulating activities (See e.g., Wasser et al., Critical Review in Immunology, 19:65-96 (1999)).

There are two major types of compounds found in *Ganoderma* which have been shown to be associated with the medicinal or therapeutic effects of *Ganoderma.* They are the polysaccharide compounds and the terpenoids. The polysaccharide compounds are primarily water-soluble. The terpenoids are oleaginous substances and are generally insoluble in water.

The polysaccharide compounds isolated from *Ganoderma* include hetero-β-glucans and their protein complexes (such as xyloglucans and acidic β-glucan-containing uronic acid, dietary fibers, lectins). The polysaccharides found in *Ganoderma* have been reported to possess anti-tumor and immune modulating effects (See Wasser et al., *supra*).

The *Ganoderma* terpenoids contain a lanostane skeleton. They are classified into several groups based on their carbon numbers and state of oxidation (Komoda et al., Chem. Pharm. Bull., 33:4829-4835 (1985)). These *Ganoderma* terpenoids include lanostanine-type triterpenoids (e.g., ganoderic acids A, B, C₁, C₂, D₁, D₂, E₁, E₂, F, G, H, I, J, K₁, K₂, L, Ma, Mb, Mc, Md, Me, Mf, Mg, Mi, Mj, Mk, Mn, N, O, P, Q, S, T, U, V, W, X, Y, and Z), 7-*O*-methyl-ganoderic acid O, trideacetyl ganoderic acid T, ganoderenic acids A, B, C, D, E, F, G, H, I, ganolucidic acids A, B, C, D, and E, lucidenic acids A, B, C, D₁, D₂, E₁, E₂, F, G, H, I, J, K, L, M, ganoderiol type I (A, B, F) and type 2 (C, D, E, F, G, H, and I), ganoderal A and B, epoxyganoderiol A, B, C, lucidone A, B, C, furanoganoderic acid, and other terpenoid components. *Ganoderma* terpenoids (e.g., ganoderic acids R, T, U-Z) have been reported to inhibit growth of hepatoma cells *in vitro* See Toth et al., Tetrahedron Lett., 24:1081-1084 (1983)).

*Ganoderma* spores are tiny mist-like brown oval-shaped spores of (6∼7) µm x (10∼12) µm in sizes which are released at the pelius of mature *Ganoderma lucidum.* These spores contain the entire genetic materials and biological substances of *Ganoderma.* However, the wild *Ganoderma* spores are difficult to collect, particularly due to their short release period, low germination rate, and low production rate under unfavorable environmental conditions. Therefore, although it is known that the *Ganoderma* spores are of greater pharmaceutical values than the fruiting bodies of *Ganoderma,* due to difficulties associated with the collection of the *Ganoderma* spores, most of thestudies on *Ganoderma* are conducted using the fruiting bodies of *Ganoderma*.

The biological substances within the *Ganoderma* spores which give rise to the therapeutic effects of *Ganoderma* are stored within the double-layered epispores of *Ganoderma lucidum.* However, these epispores have compact structure, which are extremely rigid and resilient. Therefore, it is very difficult to break-open the epispore layers of the *Ganoderma* spores and release the biological substances therein using conventional extraction methods.

There have been reports on methods for breaking the epispores of *Ganoderma* spores. For example, Japanese Patent No. JP52041208 discloses an extraction method for breaking *Ganoderma* spores using mechanical force. Chinese Patent No. CN1134306 teaches a method for breaking the sporoderm of the *Ganoderma* spores by soaking the spores in water, followed by microwave-heating. Chinese Patent No. CN1165032 teaches a method for breaking the cell wall of *Ganoderma lucidum* spores by digesting the spores with skin-dissolving enzymes such as lysozyme, snail enzyme, cellulase, or hemicellulase, followed by ultrasonic breakage of the cell walls at 20-50 C.

However, these methods use a mixed batch of spores collected from different stages of the *Ganoderma* lifecycle. It is known that the spores at different stages of the lifecycle produce different kinds and/or proportions of the biological substances, which may or may not possess the high level of therapeutic effects as expected. Therefore, the sporoderm-broken spores produced by these methods display inconsistent results and their respective medicinal effects vary.

There have also been reports on isolation or separation of the oleaginous substances (e.g., the terpenoids) from *Ganoderma*, most involving the use of organic solvents. For example, Min et al., Chem. Pharm. Bull., *supra,* disclose the isolation of lanostane-type triterpenes using column chromatography of a CHCl₃-soluble fraction of the methanol extract of the *Ganoderma* spores. Lin et al., J. Chromatography, 410: 195-200 (1987) disclose the separation of oxygenated triterpenoids from *Ganoderma lucidum* by high-performance liquid chromatography of a methanolic extract of *Ganoderma lucidum.* These methods are unsatisfactory due to complex extraction procedures and low yield of the oleaginous substances.

Derwent AN 2000-639170 discloses a method for extracting oleaginous substances from *Ganoderma* spores comprising extracting the oleaginous substances using a super critical fluid-carbon dioxide extraction (SCF-CO₂) method to obtain *Ganoderma* spore oil and degreased *Ganoderma* spore powder.

In articles of B.S. Min et al. published in Chemical and Pharmaceutical Bulletin, Vol. 48, No. 7, pp. 1026-1033 & Vol. 46, No. 10, pp. 1607-1612, respectively, specific Lanostane-type triterpenes are isolated, wherein the triterpenes are obtained by an extraction method using organic solvents in order to yield a CHCl₃-soluble fraction.

EP 1 092 765 discloses the germination activation of *Ganoderma* spores, wherein the extraction of bioactive substances is carried out by water, alcohol or thin film condensation.

In the present invention, a method for extracting the oleaginous substances from *Ganoderma* spores is provided. The *Ganoderma* spores to be used in the present invention is germination-activated to ensure that the biological substances are maximally produced. The epispores of the germination-activated *Ganoderma* spores are broken by a mechanical means to release the biological substances. The oleaginous substances of the biological substances are separated from the rest of the substances by a supercritical fluid carbon dioxide (SCF-CO₂) extraction method. The present invention has the advantage of producing high yield of oleaginous substances from *Ganoderma* (i.e., the yield of the oleaginous substances is about 37% by weight of the entire biological substances released from *Ganoderma*). The oleaginous substances isolated based on the present method demonstrate a special fragrance of *Ganoderma*, which is an indication of non-oxidation, and are without trace of solvent residue, strange odor, and deposit.

### SUMMARY OF THE INVENTION

The present invention provides a method for extracting oleaginous substances from spores of Ganoderma lucidum according to claim 1. Preferred embodiments of the method according to the invention are defined in the sub-claims. The present invention further provides the products defined is claims 21 to 24 as well as uses as defined in claims 25 to 28.

The present invention provides methods for extracting the oleaginous substances from the sporoderm-broken *Ganoderma* spores using a supercritical fluid carbon dioxide (SCF-CO₂) extraction method. The *Ganoderma* spores are germination-activated and sporoderm-broken before extraction.

To obtain the germination-activated *Ganoderma* spores, the *Ganoderma* spores are first soaked in a nutritional solution which is suitable for inducing germination. The nutritional solution for germination purpose includes, but is not limited to, an immersed solution of *Ganoderma* fruiting body, a biotin solution, water, and an immersed solution of *Ganoderma* mycelium. The immersed solution of *Ganoderma* fruiting body or Ganoderma mycelium is preferably 0.5 to 25% by weight; the preferred biotin solution is 0.1 to 0.5% by weight. The ratio between the volume of the nutritional solution and the weight of *Ganoderma* spores is about 0.01 to 5 times. The preferred soaking time is between 10 minutes to 10 hours. The preferred soaking temperature is between 16°C and 43°C.

The germination-induced *Ganoderma* spores are activated by placing the soaked *Ganoderma* spores in a well-ventilated culture box. The preferred relative humidity is 60% to 98%. The preferred temperature is 16°C to 43°C. The preferred activation period is between 10 minutes and 24 hours.

The breakage of the epispores of the *Ganoderma* spores can be achieved by applying a mechanical means to the spores. The preferred mechanical means includes, without limitation, micronization, ultra-high-speed airstream, scissorcut/grinding, and ultra-high pressure microstream. It is optional to incubate the germination-activated *Ganoderma* spores with enzymes such as chitinase and/or cellulase to soften the cell walls of the spores before applying the mechanical means to the spores.

The extraction of oleaginous substances from the germination activated and sporoderm-broken *Ganoderma* spores is carried out by a supercritical fluid carbon dioxide (SCF-CO₂) extraction method. The method includes the steps of: (1) placing the spores in a pressure vessel; (2) contacting SCF-CO₂ with the spores in the pressure vessel; and (3) depressurizing the pressure vessel to collect the oleaginous substances from the *Ganoderma* spores. The pressure in the pressure vessel is preferably between 5 M Psia (Pa) to 60 M Pa. The temperature in the pressure vessel is preferably maintained at 32°C to 85°C. The preferred flow capacity rate of the pressure vessel is between 5 kg/h and 80 kg/h. The preferred extraction time is between 30 minutes and 6 hours.

Optionally, the sporoderm-broken *Ganoderma* spores are mixed with a carrier, such as water or 85% to 100% ethanol, before being placed in the pressure vessel. The preferred ratio of the carrier to the *Ganoderma* spores is 2% to 200% by weight. The oleaginous substances are preferred to be separated from the carrier by centrifugation.

The SCF-CO₂ extraction method produces oleaginous substances from the *Ganoderma* spores, which are about 37% of the total weight of the *Ganoderma* spores. The oleaginous substances are transparent and contain a special fragrance of the *Ganoderma* spores. There is no trace of deposit, solvent residue, or oxidization in the oleaginous substances.

The oleaginous substances extracted from the sporoderm-broken *Ganoderma* spores possess medicinal effects, which include, without limitation, anti-tumor, anti-HIV or -HBV, and anti-immunological disorders. They can be used in treating patients with tumors, HIV or HBV infection, and immunological disorders.

### DETAILED DESCRIPTION OF THE INVENTION

The present invention provides an extraction method to isolate and separate the oleaginous substances from *Ganoderma* spores by using an SCF-CO₂. The amount of the oleaginous substances produced by this method constituted about 37% of the total weight of the spores. In addition, the oleaginous substances produced by this method were well preserved in its natural state (i.e., free of solvent residue and strange odor). The strange odor is an indication that the oleaginous substances have been oxidized.

Conventionally, the ways to extract or separate substances in a mixture include distillation and solvent extraction. Distillation separates the substances in a mixture according to the boiling characteristics of each substance. Solvent extraction separates the substances in a mixture according to the hydrophilic and lipophilic property of each substance. Distillation cannot be used when the substances to be separated are thermally unstable. Solvent extraction has limited utility when the substances to be separated are so similar in solubility that efficient separation cannot be obtained.

Supercritical fluids (SCFs) technology is a viable alternative to the conventional extraction methods. SCFs are often referred to as dense gases. Technically, an SCF is a gas existing above its critical temperature and critical pressure. When a gas is compressed above its critical temperature, densities increase dramatically. Therefore, under a given set of conditions, an SCF may possess the density of a liquid while maintaining the diffusivity of a gas.

Each gas has a critical pressure (Pc) and a critical temperature (Tc), above each of which a supercritical fluid state is attained. Solvent properties of such SCFs have been found to be a complex function of the fluid density, which in turn is a complex function of temperature and pressure. Thus, by varying the temperature and pressure of a supercritical fluid, extractions and precipitations can be carried out.

Carbon dioxide has proven to be a particularly advantageous gas to use in SCF extractions because it possesses good solvent properties and has low chemical reactivity and toxicity. In addition, carbon dioxide is not flammable, is inexpensive and may be readily recycled, and leaves no undesirable residues in the precipitates. Carbon dioxide has a Pc of 73.8 bar, a Tc of 31.1°C, and a density at the Pc and Tc of 0.468 g/cc.

By use of SCF-CO₂, any material in a mixture which exists in or which can be converted to a physical state that is permeable to the carbon dioxide under supercritical conditions will be dissolved in the carbon dioxide and be separated from the mixture. The principle behind the SCF-CO₂ extraction method is that under the high pressures required for extraction with gases in the supercritical fluid, the solubility of many organic compounds is increased. This, combined with the greater diffusivity of supercritical fluid over conventional solvents, results in a more rapid mass transfer through the material to be extracted, and thus a faster rate of extraction. Supercritical fluid gases have the ability to selectively dissolve and extract organic species from organic mixtures, organic/aqueous mixtures, organic/inorganic matrices, and lipophilic/hydrophilic matrices. Theoretically, the higher the pressure, the greater the efficiency of the extraction. Essentially, most or all of the oleaginous substances (with low solubility in water) in the *Ganoderma* spores should be dissolved in the carbon dioxide.

The tiny spores of *Ganoderma lucidum* has an extremely hard and resilient, double-layered epispore. In the wild, the germination of the spores of *Ganoderma lucidum* is relatively slow and their germination rate is extremely low. It takes about 24 to 48 hours for the germ tubes of the spores start to sprout under proper conditions, and the capillitia start to form branches after 72 hours, with a germination rate of only 3-15%.

When the tiny spores of *Ganoderma lucidum* were extracted under SCF-CO₂, only approximately 3.5% of the oleaginous substances were recovered.

In order to maximize the production of oleaginous substances from *Ganoderma lucidum,* a sporoderm-breaking process to break open the cell walls of the Ganoderma epispores and a germination-activation procedure were designed. This procedure was followed by a sporoderm-breaking process to break open the cell walls of the *Ganoderma* epispores. Finally, the germination-activated, sporoderm-broken *Ganoderma* spores were extracted under the SCF-CO₂ to separate the oleaginous substances from the spores.

It was noted that when the dormant *Ganoderma* spores were germination-activated, the production rate of the biological substances in the spores reached the maximum. These biological substances contain, *inter alia,* active genes and promoters, active enzymes, sterols, cytokines, interferons, lactone A, ganoderma acid A, triterpenes, polysaccharides, vitamins, superoxide dismutases (SOD), glycoproteins, etc. These biological substances demonstrate superb medicinal effects, particularly on stimulating and modulating the nervous system and the immune system. These biological substances also demonstrated therapeutic effects on liver cancer and HBV infection. Also, it was noted that during the germination-activation period, the resilience of the epispore significantly decreased, which in turn increased the penetration rate of the cell walls of the epispore.

The results of the animal and clinical studies on the effect of germination-activation showed that the biological substances produced from the germination-activated *Ganoderma* spores demonstrated inhibitory effects on liver cancer by suppressing the activity of telomerase in the hepatic cancerous tissue. These biological substances also demonstrated therapeutic effects on HBV infection. Additionally, when the germination-activated *Ganoderma* spores were given to animals, the results showed that the sporoderm-unbroken spores had an anti-tumor rate of 23.2%, which was substantially lower than the sporoderm-broken spores, which had an anti-tumor rate of 86.1%.

The extraction method of the present invention is described as follows:
1. Collection of *Ganoderma* Spores. Mature and plump *Ganoderma* spores were collected at the appropriate release time from *Ganoderma lucidum* cultured on log. It was advantageous to culture *Ganoderma* on log, because the spores thus produced were fresher and more nutritious and the penetration/breaking rate for the epispores was much higher.
2. Induction of *Ganoderma* Spores Germination. After the *Ganoderma* spores were collected, the spores could be directly broken by a mechanical means as in step 4 below and further extracted. Alternatively, the spores were induced for germination and activation as in steps 2 and 3 before the spores were broken by a mechanical force. The selected spores were soaked in a nutritional solution which could be distilled water, a saline solution, a solution which had been immersed with the fruiting bodies of *Ganoderma* or the mycelia of *Ganoderma.* The purpose of soaking the spores in the nutritional solution was to enable and accelerate the germination of the spores. Examples of the nutritional solution include 0.5∼25% by weight of the immersion solution of the *Ganoderma* fruiting bodies or mycelia, 0.1∼0.5% by weight of the biotin solution, etc. The nutritional solution was about 0.01∼5 times of the weight of the *Ganoderma* spores. The soaking time was about 10 minutes∼8 hours. The temperature was about 16∼43°C.
3. Activation of the Germinated *Ganoderma* Spores. To activate the germinated *Ganoderma* spores, the soaked spores were removed from the nutritional solution and excess solution was allowed to drip. The soaked spores were then placed in a well-ventilated culture box which was kept in constant temperature and humidity. The relative humidity in the culture box was maintained at about 60∼98%. The temperature of the culture box was maintained at about 16∼48°C. The time for activating the spores was about 10 minutes ∼ 24 hours.
4. Penetration/Breakage of the Epispores. After the *Ganoderma* spores were germination-activated, the spores were further broken by a mechanical means. Examples of the mechanical means used to break the spores include micronization, roll-pressing, or scissor-cut/grinding, microstream-impact crushing, ultra-high-speed airstream impact crushing, ultra-high pressure microstream crushing, ultra-low temperature crushing etc.
   Before breaking the epispores, it was optional to treat the spores with enzymes such as chitinase and cellulase to soften the cell walls of the epispores. The enzyme-treated spores could be separated from the reaction mixture by centrifugation at about 3,000∼30,000 rpm or ultra-filtration using a filter with about 10,000 molecular weight cut-off.
5. Extraction of Oleaginous Substances with SCF-CO₂. The extraction of the oleaginous substances from the sporoderm-broken spores was conducted in an SCF-CO₂ extracting apparatus, which included a CO₂ source, a compressor, a heat exchanger, a pressure regulator, and a pressure vessel. Alternatively, any conventional supercritical fluid extraction equipment which contains an extractor i.e., the pressure vessel) and a separator would also be suitable for the extraction. To operate, the sporoderm-broken spores were placed in the pressure vessel. The carbon dioxide was flowed through the compressor and heat exchanger to achieve greater than supercritical temperature and pressure, and then flowed through the spores in the pressure vessel. The SCF was then removed from the pressure vessel and depressurized to evaporate the carbon dioxide. The supercritical pressure used in the present method was about 5∼60 M Pa. The supercritical temperature was in the present method was about 32∼85°C. The flow volume rate of CO₂ was about 5∼80 kg/h. The extraction time was about 0.5∼6 hours.

It was optional to add a carrier to the spores between initiating the SCF-CO₂ extraction. Examples of the carrier include water or 85 ∼ 100% of ethanol. The ratio of the carrier to the spores was about 2∼200 % (v/w). When the carrier was added to the spores, the oleaginous substances could be separated from the rest of the spores by centrifugation at about 3,000∼30,000 rpm.

### EXAMPLE 1

The oleaginous substances from the *Ganoderma* spores were extracted according to the method shown below:
1. One hundred (100) kg of mature and plump *Ganoderma* spores were carefully selected.
2. A nutritional solution containing 2 kg of 5% immersion solution of *Ganoderma* fruiting bodies and 150 kg of saline (0.15 M NaCl) was added to the spores. The spores were soaked in the nutritional solution for 6 hours at 32°C.
3. The soaked *Ganoderma* spores were removed from the nutritional solution and placed into a well-ventilated culture box which was kept at constant temperature of 34°C and relative humidity of 90%. The spores were maintained in the culture box for 3 hours. Under such condition, the germination rate of the spores was 95% and the cell walls of the germination-activated spores showed clear signs of softening.
4. The germination-activated *Ganoderma* spores were micronized. The spores were monitored under the microscope. The penetration/breaking rate of the epispores reached 99.6% after this treatment.
5. The epispore-broken Ganoderma spores were placed in the pressure vessel of the extraction apparatus for SCF-CO₂. The supercritical pressure was kept at 45 M Pa and the supercritical temperature was at 53°C. The flow volume rate of CO₂ was kept at 60 kg/h. The total extraction time was 1.5 hours.

### Results:

This method produced about 37 kg of oleaginous substances (which constituted approximately 37% by weight of the spores). The oleaginous substances were transparent and contained the special fragrance of *Ganoderma* spores, an indication that the oleaginous substances were not oxidized. There was no trace of deposit or solvent residue in the oleaginous substances.

### EXAMPLE 2

The oleaginous substances from the *Ganoderma* spores were extracted according to the method shown below:
1. One hundred (100) kg of mature and plump *Ganoderma* spores were carefully selected.
2. A nutritional solution containing 20 kg of 1% biotin solution and 250 kg of distilled water was added to the spores. The spores were soaked in the nutritional solution for 3 hours at 43°C.
3. The soaked *Ganoderma* spores were removed from the nutritional solution and placed into a well-ventilated culture box which was kept at temperature of 18°C and relative humidity of 60%. The spores were maintained in the culture box for 24 hours. Under such condition, the germination rate of the spores was 95% and the cell walls of the germination-activated spores showed clear signs of softening.
4. An enzyme mixture containing 5 g of chitinase, 10 g of cellulose, and 150 kg of distilled water (at pH 6.8) was added to and reacted with the spores. The reaction was performed at the temperature of 43 C for 1.5 hours. At the end of the reaction, the epispores had lost their resilience.
5. The spores were micronized with crushing. The spores were monitored under the microscope. The penetration/breaking rate of the epispores reached 99.6% after this treatment. The sporoderm-broken spores were separated from the enzyme mixture by centrifugation at 3,000 rpm.
6. The spores were then placed in the pressure vessel of the extraction apparatus for SCF-CO₂. Ten (10) kg of 90% ethanol were added to the spores as a carrier. The supercritical pressure was kept at 40 M Pa and the supercritical temperature was at 32°C. The flow volume rate of CO₂ was kept at 15 kg/h. The total extraction time was 5 hours.
7. After SCF-CO2 extraction, the oleaginous substances were separated from the carrier by centrifugation at 6,000 rpm.

### Results:

This method produced about 36.9 kg of oleaginous substances (which constituted approximately 36.9% by weight of the spores). The oleaginous substances were transparent and contained the special fragrance of *Ganoderma* spores, an indication that the oleaginous substances were not oxidized. There was no trace of deposit or solvent residue in the oleaginous substances.

### EXAMPLE 3

The oleaginous substances from the *Ganoderma* spores were extracted according to the method shown below:
1. One hundred (100) kg of mature and plump *Ganoderma* spores were carefully selected.
2. A nutritional solution containing 200 kg of distilled water was added to the spores. The spores were soaked in this solution for 8 hours at 16°C.
3. The soaked spores were removed from the nutritional solution and placed in a well-ventilated culture box which was kept at temperature of 48°C and relative humidity of 98% for 10 minutes. Under such condition, the germination rate of the spores was 95% and the cell walls of the germination-activated spores showed clear signs of softening.
4. An enzyme mixture containing 10 g of chitinase, 20 g of cellulose, and 200 kg of distilled water (at pH 6.8) was added to and reacted with the spores. The reaction was performed at the temperature of 38 C for 3.0 hours. At the end of the reaction, the epispores had lost their resilience.
5. The sporoderm of the spores were broken by rolling/pressing with crushing. The spores were monitored under the microscope. The sporoderm-broken spores were separated from the enzyme mixture by ultra-filtration.
6. The spores were then placed in the pressure vessel of the extraction apparatus for SCF-CO₂. The supercritical pressure was kept at 60 M Pa and the supercritical temperature was at 48°C. The flow volume rate of CO₂ was kept at 5 kg/h. The total extraction time was 4.5 hours.

### Results:

This method produced about 36.9 kg of oleaginous substances (which constituted approximately 36.9% by weight of the spores). The oleaginous substances were transparent and contained the special fragrance of *Ganoderma* spores, an indication that the oleaginous substances were not oxidized. There was no trace of deposit or solvent residue in the oleaginous substances.

### EXAMPLE 4

The oleaginous substances from the *Ganoderma* spores were extracted according to the method shown below:
1. One hundred (100) kg of mature and plump *Ganoderma* spores were carefully selected.
2. A nutritional solution containing 10 kg of 0.5% of immersed solution of *Ganoderma* fruiting bodies, 10 kg of 0.1% biotin solution, and 200 kg of distilled water was added to the spores. The spores were soaked in this solution for 30 minutes at 25°C.
3. The soaked spores were removed from the nutritional solution and placed in a well-ventilated culture box which was kept at temperature of 16°C and relative humidity of 80% for 24 hours. Under such condition, the germination rate of the spores was 95% and the cell walls of the germination-activated spores showed clear signs of softening.
4. Ultra-high-speed airstream was used to break the epispores of the spores. The broken spores were monitored under a microscope. About 99.6% of the spores were broken under this condition.
5. The spores were then placed in the pressure vessel of the extraction apparatus for SCF-CO₂. Fifty (50) kg of 100% ethanol were added as a carrier. The supercritical pressure was kept at 10 M Pa and the supercritical temperature was at 85°C. The flow volume rate of CO₂ was kept at 80 kg/h. The total extraction time was 6 hours.
6. After SCF-CO₂ extraction, the oleaginous substances were separated from the carrier by centrifugation at 20,000 rpm.

### Results:

This method produced about 37 kg of oleaginous substances (which constituted approximately 37% by weight of the spores). The oleaginous substances were transparent and contained the special fragrance of *Ganoderma* spores, an indication that the oleaginous substances were not oxidized. There was no trace of deposit or solvent residue in the oleaginous substances.

### EXAMPLE 5

The oleaginous substances from the *Ganoderma* spores were extracted according to the method shown below:
1. One hundred (100) kg of mature and plump *Ganoderma* spores were carefully selected.
2. A nutritional solution containing 50 kg of 5% of immersed solution of *Ganoderma* mycelia and 5 kg of 0.1% biotin solution was added to the spores. The spores were soaked in this solution for 5 hours at 38°C.
3. The soaked spores were removed from the nutritional solution and placed in a well-ventilated culture box which was kept at temperature of 30°C and relative humidity of 70% for 1.5 hours. Under such condition, the germination rate of the spores was 95% and the cell walls of the germination-activated spores showed clear signs of softening.
4. An enzyme mixture containing 20 g of chitinase and 180 kg of distilled water (at pH 6.4) was added to and reacted with the spores. The reaction was performed at the temperature of 38 Cfor 4.0 hours. At the end of the reaction, the epispores had lost their resilience.
5. The spores were broken by scissor-cutting/ grinding. The broken spores were monitored under a microscope. About 99.6% of the spores were broken under this condition. The sporoderm-broken spores were separated from the enzyme mixture by centrifugation at 15,000 rpm.
6. The spores were then placed in the pressure vessel of the extraction apparatus for SCF-CO₂. Fifty (50) kg of distilled water were added as a carrier. The supercritical pressure was kept at 55 M Pa and the supercritical temperature was at 55°C. The flow volume rate of CO₂ was kept at 42 kg/h. The total extraction time was 2 hours.
7. After SCF-CO₂ extraction, the oleaginous substances were separated from the carrier by centrifugation at 20,000 rpm.

### Results:

This method produced about 37 kg of oleaginous substances (which constituted approximately 37% by weight of the spores). The oleaginous substances were transparent and contained the special fragrance of *Ganoderma* spores, an indication that the oleaginous substances were not oxidized. There was no trace of deposit or solvent residue in the oleaginous substances.

### EXAMPLE 6

The oleaginous substances from the *Ganoderma* spores were extracted according to the method shown below:
1. One hundred (100) kg of mature and plump *Ganoderma* spores were carefully selected.
2. A nutritional solution containing 5 kg of 20% of immersed solution of *Ganoderma* fruiting bodies and 150 kg of distilled water was added to the spores. The spores were soaked in this solution for 2 hours at 28°C.
3. The soaked spores were removed from the nutritional solution and placed in a well-ventilated culture box which was kept at temperature of 40°C and relative humidity of 65% for 3 hours. Under such condition, the germination rate of the spores was 95% and the cell walls of the germination-activated spores showed clear signs of softening.
4. The epispores of the spores were broken by micronization. The broken spores were monitored under a microscope. About 99.7% of the spores were broken under this condition.
5. The spores were then placed in the pressure vessel of the extraction apparatus for SCF-CO₂. Two hundred (200) kg of 95% ethanol were added as a carrier. The supercritical pressure was kept at 55 M Pa and the supercritical temperature was at 68°C. The flow volume rate of CO₂ was kept at 70 kg/h. The total extraction time was 2 hours.
7. After SCF-CO₂ extraction, the oleaginous substances were separated from the carrier by centrifugation at 20,000 rpm.

### Results:

This method produced about 37 kg of oleaginous substances (which constituted approximately 37% by weight of the spores). The oleaginous substances were transparent and contained the special fragrance of *Ganoderma* spores, an indication that the oleaginous substances were not oxidized. There was no trace of deposit or solvent residue in the oleaginous substances.

### EXAMPLE 7

The oleaginous substances from the *Ganoderma* spores were extracted according to the method shown below:
1. One hundred (100) kg of mature and plump *Ganoderma* spores were carefully selected.
2. A nutritional solution containing 20 kg of 25% of immersed solution of *Ganoderma* mycelia and 150 kg of distilled water was added to the spores. The spores were soaked in this solution for 6 hours at 22°C.
3. The soaked spores were removed from the nutritional solution and placed in a well-ventilated culture box which was kept at temperature of 26°C and relative humidity of 75% for 4 hours. Under such condition, the germination rate of the spores was 95% and the cell walls of the germination-activated spores showed clear signs of softening.
4. An enzyme mixture containing 20 g of cellulose and 250 kg of distilled water (at pH 5.6) was added to and reacted with the spores. The reaction was performed at the temperature of 45 C for 2 hours. At the end of the reaction, the epispores had lost their resilience.
5. The spores were broken by rolling/pressing. The broken spores were monitored under a microscope. About 99.7% of the spores were broken under this condition. The sporoderm-broken spores were separated from the enzyme mixture by centrifugation at 15,000 rpm.
6. The spores were then placed in the pressure vessel of the extraction apparatus for SCF-CO₂. The supercritical pressure was kept at 55 M Pa and the supercritical temperature was at 70°C. The flow volume rate of CO₂ was kept at 60 kg/h. The total extraction time was 0.5 hours.

### Results:

This method produced about 36.9 kg of oleaginous substances (which constituted approximately 36.9% by weight of the spores). The oleaginous substances were transparent and contained the special fragrance of *Ganoderma* spores, an indication that the oleaginous substances were not oxidized. There was no trace of deposit or solvent residue in the oleaginous substances.

### EXAMPLE 8

The oleaginous substances from the *Ganoderma* spores were extracted according to the method shown below:
1. One hundred (100) kg of mature and plump *Ganoderma* spores were carefully selected.
2. A nutritional solution containing 20 kg of 1% of biotin solution and 150 kg of distilled water was added to the spores. The spores were soaked in this solution for 2 hours at 40°C.
3. The soaked spores were removed from the nutritional solution and placed in a well-ventilated culture box which was kept at temperature of 18°C and relative humidity of 88% for 12 hours. Under such condition, the germination rate of the spores was 95% and the cell walls of the germination-activated spores showed clear signs of softening.
4. The epispores of the spores were broken by scissor-cut/grinding. The broken spores were monitored under a microscope. About 99.9% of the spores were broken under this condition.
5. The spores were then placed in the pressure vessel of the extraction apparatus for SCF-CO₂. Seventy (70) kg of 95% ethanol were added as a carrier. The supercritical pressure was kept at 10 M Pa and the supercritical temperature was at 38°C. The flow volume rate of CO₂ was kept at 18 kg/h. The total extraction time was 4 hours.
6. After SCF-CO₂ extraction, the oleaginous substances were separated from the carrier by centrifugation at 15,000 rpm.

### Results:

This method produced about 37 kg of oleaginous substances (which constituted approximately 37% by weight of the spores). The oleaginous substances were transparent and contained the special fragrance of *Ganoderma* spores, an indication that the oleaginous substances were not oxidized. There was no trace of deposit or solvent residue in the oleaginous substances.

### EXAMPLE 9

The oleaginous substances from the *Ganoderma* spores were extracted according to the method shown below:
1. One hundred (100) kg of mature and plump *Ganoderma* spores were carefully selected.
2. A nutritional solution containing 250 kg of distilled water was added to the spores. The spores were soaked in this solution for 10 hours at 34°C.
3. The soaked spores were removed from the nutritional solution and placed in a well-ventilated culture box which was kept at temperature of 31°C and relative humidity of 80% for 2 hours. Under such condition, the germination rate of the spores was 95% and the cell walls of the germination-activated spores showed clear signs of softening.
4. An enzyme mixture containing 10 g of chitinase and 250 kg of distilled water (at pH 6.8) was added to and reacted with the spores. The reaction was performed at the temperature of 44 Cfor 2 hours. At the end of the reaction, the epispores had lost their resilience.
5. The spores were broken by micronization/pressing. The broken spores were monitored under a microscope. About 99.9% of the spores were broken under this condition. The sporoderm-broken spores were separated from the enzyme mixture by centrifugation at 30,000 rpm.
6. The spores were then placed in the pressure vessel of the extraction apparatus for SCF-CO₂. Two (2) kg of distilled water were added as a carrier. The supercritical pressure was kept at 14 M Pa and the supercritical temperature was at 45°C. The flow volume rate of CO₂ was kept at 12 kg/h. The total extraction time was 2 hours.
7. After SCF-CO₂ extraction, the oleaginous substances were separated from the carrier by centrifugation at 30,000 rpm.

### Results:

This method produced about 36.8 kg of oleaginous substances (which constituted approximately 36.8% by weight of the spores). The oleaginous substances were transparent and contained the special fragrance of *Ganoderma* spores, an indication that the oleaginous substances were not oxidized. There was no trace of deposit or solvent residue in the oleaginous substances.

### EXAMPLE 10

The oleaginous substances from the *Ganoderma* spores were extracted according to the method shown below:
1. One hundred (100) kg of mature and plump *Ganoderma* spores were carefully selected.
2. A nutritional solution containing 200 kg of saline solution was added to the spores. The spores were soaked in this solution for 6 hours at 22°C.
3. The soaked spores were removed from the nutritional solution and placed in a well-ventilated culture box which was kept at temperature of 34°C and relative humidity of 75% for 5 hours. Under such condition, the germination rate of the spores was 95% and the cell walls of the germination-activated spores showed clear signs of softening.
4. The epispores of the spores were broken by ultra-high pressure microstream. The broken spores were monitored under a microscope. About 99.9% of the spores were broken under this condition.
5. The spores were then placed in the pressure vessel of the extraction apparatus for SCF-CO₂. Sixty five (65) kg of 85% ethanol were added as a carrier. The supercritical pressure was kept at 14 M Pa and the supercritical temperature was at 40°C. The flow volume rate of CO₂ was kept at 25 kg/h. The total extraction time was 1 hour.
6. After SCF-CO₂ extraction, the oleaginous substances were separated from the carrier by centrifugation at 8,000 rpm.

### Results:

This method produced about 37 kg of oleaginous substances (which constituted approximately 37% by weight of the spores). The oleaginous substances were transparent and contained the special fragrance of *Ganoderma* spores, an indication that the oleaginous substances were not oxidized. There was no trace of deposit or solvent residue in the oleaginous substances.

### EXAMPLE 11

The oleaginous substances from the *Ganoderma* spores were extracted according to the method shown below:
1. One hundred (100) kg of mature and plump *Ganoderma* spores were carefully selected.
2. The *Ganoderma* spores were micronized. The spores were monitored under the microscope. The penetration/breaking rate of the epispores reached 99.6% after this treatment.
3. The epispore-broken Ganoderma spores were placed in the pressure vessel of the extraction apparatus for SCF-CO₂. The supercritical pressure was kept at 35 M Pa and the supercritical temperature was at 50°C. The flow volume rate of CO₂ was kept at 25 kg/h. The total extraction time was 3.0 hours.

### Results:

This method produced about 37 kg of oleaginous substances (which constituted approximately 37% by weight of the spores). The oleaginous substances were transparent and contained the special fragrance of *Ganoderma* spores, an indication that the oleaginous substances were not oxidized. There was no trace of deposit or solvent residue in the oleaginous substances.

### EXAMPLE 12

The oleaginous substances from the *Ganoderma* spores were extracted according to the method shown below:
1. One hundred (100) kg of mature and plump *Ganoderma* spores were carefully selected.
2. An enzyme mixture containing 10 g of chitinase, 20 g of cellulose, and 200 kg of distilled water (at pH 6.2) was added to and reacted with the spores. The reaction was performed at the temperature of 45 C for 1 hour. At the end of the reaction, the epispores had lost their resilience.
3. The spores were micronized with crushing. The spores were monitored under the microscope. The penetration/breaking rate of the epispores reached 99.9% after this treatment. The sporoderm-broken spores were separated from the enzyme mixture by centrifugation at 3,000 rpm.
4. The spores were then placed in the pressure vessel of the extraction apparatus for SCF-CO₂. Ten (10) kg of 90% ethanol were added to the spores as a carrier. The supercritical pressure was kept at 30 M Pa and the supercritical temperature was at 41°C. The flow volume rate of CO₂ was kept at 5 kg/h. The total extraction time was 5 hours.
5. After SCF-CO₂ extraction, the oleaginous substances were separated from the carrier by centrifugation at 3,000 rpm.

### Results:

This method produced about 37.1 kg of oleaginous substances (which constituted approximately 37.1% by weight of the spores). The oleaginous substances were transparent and contained the special fragrance of *Ganoderma* spores, an indication that the oleaginous substances were not oxidized. There was no trace of deposit or solvent residue in the oleaginous substances.

### EXAMPLE 13

The oleaginous substances from the *Ganoderma* spores were extracted according to the method shown below:
1. One hundred (100) kg of mature and plump *Ganoderma* spores were carefully selected.
2. An enzyme mixture containing 20 g of chitinase, 10 g of cellulose, and 250 kg of distilled water (at pH 6.5) was added to and reacted with the spores. The reaction was performed at the temperature of 48 Cfor 2 hours. At the end of the reaction, the epispores had lost their resilience.
3. The sporoderm of the spores were broken by rolling/pressing with crushing. The spores were monitored under the microscope. The sporoderm-broken spores were separated from the enzyme mixture by ultra-filtration.
4. The spores were then placed in the pressure vessel of the extraction apparatus for SCF-CO₂. The supercritical pressure was kept at 40 M Pa and the supercritical temperature was at 32°C. The flow volume rate of CO₂ was kept at 10 kg/h. The total extraction time was 4 hours.

### Results:

This method produced about 37.3 kg of oleaginous substances (which constituted approximately 37.3% by weight of the spores). The oleaginous substances were transparent and contained the special fragrance of *Ganoderma* spores, an indication that the oleaginous substances were not oxidized. There was no trace of deposit or solvent residue in the oleaginous substances.

### EXAMPLE 14

The oleaginous substances from the *Ganoderma* spores were extracted according to the method shown below:
1. One hundred (100) kg of mature and plump *Ganoderma* spores were carefully selected.
2. Ultra-high-speed airstream was used to break the epispores of the spores. The broken spores were monitored under a microscope. About 99.6% of the spores were broken under this condition.
3. The spores were then placed in the pressure vessel of the extraction apparatus for SCF-CO₂. Twenty (20) kg of 100% ethanol were added as a carrier. The supercritical pressure was kept at 10 M Pa and the supercritical temperature was at 35°C. The flow volume rate of CO₂ was kept at 12 kg/h. The total extraction time was 6 hours.
4. After SCF-CO₂ extraction, the oleaginous substances were separated from the carrier by centrifugation at 5,000 rpm.

### Results:

This method produced about 37.2 kg of oleaginous substances (which constituted approximately 37.2% by weight of the spores). The oleaginous substances were transparent and contained the special fragrance of *Ganoderma* spores, an indication that the oleaginous substances were not oxidized. There was no trace of deposit or solvent residue in the oleaginous substances.

### EXAMPLE 15

The oleaginous substances from the *Ganoderma* spores were extracted according to the method shown below:
1. One hundred (100) kg of mature and plump *Ganoderma* spores were carefully selected.
2. An enzyme mixture containing 30 g of chitinase and 250 kg of distilled water (at pH 6.2) was added to and reacted with the spores. The reaction was performed at the temperature of 45 C for 2.5 hours. At the end of the reaction, the epispores had lost their resilience.
3. The spores were broken by scissor-cutting/ grinding. The broken spores were monitored under a microscope. About 99.9% of the spores were broken under this condition. The sporoderm-broken spores were separated from the enzyme mixture by centrifugation at 15,000 rpm.
4. The spores were then placed in the pressure vessel of the extraction apparatus for SCF-CO₂. Fifty (50) kg of distilled water were added as a carrier. The supercritical pressure was kept at 50 M Pa and the supercritical temperature was at 60°C. The flow volume rate of CO₂ was kept at 40 kg/h. The total extraction time was 2.5 hours.
5. After SCF-CO₂ extraction, the oleaginous substances were separated from the carrier by centrifugation at 10,000 rpm.

### Results:

This method produced about 37.6 kg of oleaginous substances (which constituted approximately 37.6% by weight of the spores). The oleaginous substances were transparent and contained the special fragrance of *Ganoderma* spores, an indication that the oleaginous substances were not oxidized. There was no trace of deposit or solvent residue in the oleaginous substances.

### EXAMPLE 16

The oleaginous substances from the *Ganoderma* spores were extracted according to the method shown below:
1. One hundred (100) kg of mature and plump *Ganoderma* spores were carefully selected.
2. The epispores of the spores were broken by micronization. The broken spores were monitored under a microscope. About 99.7% of the spores were broken under this condition.
3. The spores were then placed in the pressure vessel of the extraction apparatus for SCF-CO₂. One hundred (100) kg of 95% ethanol were added as a carrier. The supercritical pressure was kept at 55 M Pa and the supercritical temperature was at 68°C. The flow volume rate of CO₂ was kept at 70 kg/h. The total extraction time was 2 hours.
4. After SCF-CO₂ extraction, the oleaginous substances were separated from the carrier by centrifugation at 20,000 rpm.

### Results:

This method produced about 37.5 kg of oleaginous substances (which constituted approximately 37.5% by weight of the spores). The oleaginous substances were transparent and contained the special fragrance of *Ganoderma* spores, an indication that the oleaginous substances were not oxidized. There was no trace of deposit or solvent residue in the oleaginous substances.

### EXAMPLE 17

The oleaginous substances from the *Ganoderma* spores were extracted according to the method shown below:
1. One hundred (100) kg of mature and plump *Ganoderma* spores were carefully selected.
2. An enzyme mixture containing 30 g of cellulose and 200 kg of distilled water (at pH 5.8) was added to and reacted with the spores. The reaction was performed at the temperature of 40 C for 1.5 hours. At the end of the reaction, the epispores had lost their resilience.
3. The spores were broken by rolling/pressing. The broken spores were monitored under a microscope. About 99.8% of the spores were broken under this condition. The sporoderm-broken spores were separated from the enzyme mixture by centrifugation at 20,000 rpm.
6. The spores were then placed in the pressure vessel of the extraction apparatus for SCF-CO₂. The supercritical pressure was kept at 60 M Pa and the supercritical temperature was at 75°C. The flow volume rate of CO₂ was kept at 80 kg/h. The total extraction time was 0.5 hours.

### Results:

This method produced about 37.4 kg of oleaginous substances (which constituted approximately 37.4% by weight of the spores). The oleaginous substances were transparent and contained the special fragrance of *Ganoderma* spores, an indication that the oleaginous substances were not oxidized. There was no trace of deposit or solvent residue in the oleaginous substances.

### EXAMPLE 18

The oleaginous substances from the *Ganoderma* spores were extracted according to the method shown below:
1. One hundred (100) kg of mature and plump *Ganoderma* spores were carefully selected.
2. The epispores of the spores were broken by scissor-cut/grinding. The broken spores were monitored under a microscope. About 99.9% of the spores were broken under this condition.
3. The spores were then placed in the pressure vessel of the extraction apparatus for SCF-CO₂. One hundred (100) kg of 90% ethanol were added as a carrier. The supercritical pressure was kept at 15 M Pa and the supercritical temperature was at 48°C. The flow volume rate of CO₂ was kept at 25 kg/h. The total extraction time was 2 hours.
4. After SCF-CO₂ extraction, the oleaginous substances were separated from the carrier by centrifugation at 25,000 rpm.

### Results:

This method produced about 37.5 kg of oleaginous substances (which constituted approximately 37.5% by weight of the spores). The oleaginous substances were transparent and contained the special fragrance of *Ganoderma* spores, an indication that the oleaginous substances were not oxidized. There was no trace of deposit or solvent residue in the oleaginous substances.

### EXAMPLE 19

The oleaginous substances from the *Ganoderma* spores were extracted according to the method shown below:
1. One hundred (100) kg of mature and plump *Ganoderma* spores were carefully selected.
2. An enzyme mixture containing 10 g of chitinase, 10 kg of cellulase and 220 kg of distilled water (at pH 7.3) was added to and reacted with the spores. The reaction was performed at the temperature of 45 Cfor 1.5 hours. At the end of the reaction, the epispores had lost their resilience.
3. The spores were broken by micronization/pressing. The broken spores were monitored under a microscope. About 99.9% of the spores were broken under this condition. The sporoderm-broken spores were separated from the enzyme mixture by ultra-filtration.
4. The spores were then placed in the pressure vessel of the extraction apparatus for SCF-CO₂. Ten (10) kg of distilled water were added as a carrier. The supercritical pressure was kept at 5 M Pa and the supercritical temperature was at 85°C. The flow volume rate of CO₂ was kept at 35 kg/h. The total extraction time was 1.5 hours.
5. After SCF-CO₂ extraction, the oleaginous substances were separated from the carrier by centrifugation at 30,000 rpm.

### Results:

This method produced about 37.1 kg of oleaginous substances (which constituted approximately 37.1% by weight of the spores). The oleaginous substances were transparent and contained the special fragrance of *Ganoderma* spores, an indication that the oleaginous substances were not oxidized. There was no trace of deposit or solvent residue in the oleaginous substances.

### EXAMPLE 20

The oleaginous substances from the *Ganoderma* spores were extracted according to the method shown below:
1. One hundred (100) kg of mature and plump *Ganoderma* spores were carefully selected.
2. The epispores of the spores were broken by ultra-high pressure microstream. The broken spores were monitored under a microscope. About 99.9% of the spores were broken under this condition.
5. The spores were then placed in the pressure vessel of the extraction apparatus for SCF-CO₂. Two hundred (200) kg of 80% ethanol were added as a carrier. The supercritical pressure was kept at 14 M Pa and the supercritical temperature was at 40°C. The flow volume rate of CO₂ was kept at 12 kg/h. The total extraction time was 0.5 hour.
6. After SCF-CO₂ extraction, the oleaginous substances were separated from the carrier by centrifugation at 6,000 rpm.

### Results:

This method produced about 37.4 kg of oleaginous substances (which constituted approximately 37.4% by weight of the spores). The oleaginous substances were transparent and contained the special fragrance of *Ganoderma* spores, an indication that the oleaginous substances were not oxidized. There was no trace of deposit or solvent residue in the oleaginous substances.

## Claims

1. A method for extracting oleaginous substances from spores of *Ganoderma lucidum* comprising:
germination-activating said Ganoderma spores;
breaking said germination-activated *Ganoderma* spores by a mechanical means to obtain sporoderm-broken spores; and
extracting said oleaginous substances from said sporoderm-broken spores using a supercritical fluid - carbon dioxide (SCF-CO₂) extraction method.

2. The method according to claim 1, wherein before said *Ganoderma* spores are broken by said mechanical means, said *Ganoderma* spores are germination-activated by soaking said *Ganoderma* spores in a nutritional solution which is suitable for induction of germination; and placing said germination-induced *Ganoderma* spores in a ventilated culture box for activation until cell walls of said *Ganoderma* spores are softened.

3. The method according to claim 2, wherein said nutritional solution is at least one which is selected from the group consisting of an immersed solution of *Ganoderma* fruiting body, a biotin solution, water, and an immersed solution of *Ganoderma* mycelium.

4. The method according to claim 2, wherein said *Ganoderma* spores are soaked in said nutritional solution between 10 minutes and 10 hours.

5. The method according to claim 2, wherein said *Ganoderma* spores are soaked in said nutritional solution between 16° and 43°C.

6. The method according to claim 2, wherein said ventilated culture box is at relative humidity between 60% and 98%.

7. The method according to claim 2, wherein said ventilated culture box is at temperature between 16°C and 48°C.

8. The method according to claim 2, wherein said soaked *Ganoderma* spores are placed in said ventilated culture box between 10 minutes and 24 hours.

9. The method according to claim 1, wherein said mechanical means is at least one selected from the group consisting of micronization, ultra-high-speed airstream, scissor-cut/grinding, and ultra-high pressure microstream.

10. The method according to claim 1, further comprising a step of digesting said *Ganoderma* spores with at least an enzyme before applying said mechanical means.

11. The method according to claim 10, wherein said enzyme is at least one selected from the group consisting of chitinase and cellulase.

12. The method according to claim 1, wherein said SCF-CO₂ extraction method comprises:
placing said sporoderm-broken Ganoderma spores in a pressure vessel;
contacting SCF-CO₂ with said Ganoderma spores in said pressure vessel; and
depressurizing said pressure vessel to collect said oleaginous substances from said sporoderm-broken *Ganoderma* spores.

13. The method according to claim 12, wherein said pressure vessel is maintained at a pressure between 5 M Psia (Pa) to 60 M Pa.

14. The method according to claim 12, wherein said pressure vessel is maintained at a temperature of 32°C to 85°C.

15. The method according to claim 12, wherein said pressure vessel is maintained at a flow volume rate of 5 kg/h to 80 kg/h.

16. The method according to claim 12, wherein said extraction time is between 30 minutes and 6 hours.

17. The method according to claim 12, wherein said sporoderm-broken *Ganoderma* spores are mixed with a carrier before placed in said pressure vessel.

18. The method according to claim 17, wherein said carrier is 85% to 100% ethanol (vol/vol) or water.

19. The method according to claim 17, wherein said carrier and said *Ganoderma* spores are at a weight ratio of 2% to 200%.

20. The method according to claim 17, further comprising a step of:
separating said oleaginous substances from said carrier by centrifugation.

21. An anti-tumor agent comprising said oleaginous substances from *Ganoderma* spores obtainable according to claim 1.

22. An anti-HIV agent comprising said oleaginous substances from *Ganoderma* spores obtainable according to claim 1.

23. An anti-HBV agent comprising said oleaginous substances from *Ganoderma* spores obtainable according to claim 1.

24. An immune modulating agent comprising said oleaginous substances from *Ganoderma* spores obtainable according to claim 1.

25. A use of an effective amount of said oleaginous substances from *Ganoderma* spores obtainable according to claim 1 for the preparation of a medicament for treating patient with tumors.

26. A use of an effective amount of said oleaginous substances from *Ganoderma* spores obtainable according to claim 1 for the preparation of a medicament for treating patient with HIV infection.

27. A use of an effective amount of said oleaginous substances from *Ganoderma* spores obtainable according to claim 1 for the preparation of a medicament for trea patient with HBV infection.

28. A use of an effective amount of said oleaginous substances from *Ganoderma* spores obtainable according to claim 1 for the preparation of a medicament for treating patient with immunological disorders.

## Patentansprüche

1. Verfahren zum Extrahieren öliger Substanzen aus Sporen von *Ganoderma lucidum*, umfassend die Schritte:
Keimungs-Aktivierung der *Ganoderma*-Sporen;
Aufbrechen der besagten keimungs-aktivierten *Ganoderma*-Sporen durch ein mechanisches Mittel, zum Erhalten von sporoderm-aufgebrochenen Sporen; und
Extrahieren der öligen Substanzen aus den sporoderm-aufgebrochenen Sporen unter Verwendung eines superkritischen Kohlendioxyd-Fluid-(SCF-CO₂)-Extraktionsverfahrens.

2. Verfahren gemäß Anspruch 1, wobei, bevor die *Ganoderma*-Sporen durch das mechanische Mittel aufgebrochen werden, die *Ganoderma*-Sporen durch Eintauchen der Ganoderma-Sporen in eine Nährlösung keimungs-aktiviert werden, die zur Induktion der Keimung geeignet ist; und Einbringen der keimungsinduzierten *Ganoderma*-Sporen in eine belüftete Kulturbox zur Aktivierung, bis die Zellwände der *Ganoderma*-Sporen erweicht sind.

3. Verfahren gemäß Anspruch 2, wobei die Nährlösung mindestens eine ist, die aus der Gruppe ausgewählt wird, die aus einer Eintauchlösung von *Ganoderma*-Fruchtkörpern, einer Biotinlösung, Wasser und einer Eintauchlösung von *Ganoderma*-Mycelium besteht.

4. Verfahren gemäß Anspruch 2, wobei die *Ganoderma*-Sporen 10 Minuten bis 10 Stunden in die Nährlösung eingetaucht werden.

5. Verfahren gemäß Anspruch 2, wobei die *Ganoderma*-Sporen in die Nährlösung bei 16°C bis 43°C eingetaucht werden.

6. Verfahren gemäß Anspruch 2, wobei die belüftete Kulturbox bei einer relativen Luftfeuchtigkeit von 60 % bis 98 % ist.

7. Verfahren gemäß Anspruch 2, wobei die belüftete Kulturbox bei einer Temperatur von 16°C bis 48°C ist.

8. Verfahren gemäß Anspruch 2, wobei die eingetauchten *Ga noderma*-Sporen 10 Minuten bis 24 Stunden in die belüftete Kulturbox eingebracht werden.

9. Verfahren gemäß Anspruch 1, wobei das mechanische Mittel mindestens eines ist das aus der Gruppe ausgewählt wird, die aus Mikronisierung, Luftstrom ultrahoher Geschwindigkeit, Zerschneiden/Zerkleinern und Mikrostrom ultrahohen Drucks besteht.

10. Verfahren gemäß Anspruch 1, ferner umfassend einen Schritt des Verdauens der *Ganoderma*-Sporen mit mindestens einem Enzym vor dem Anwenden des mechanischen Mittels.

11. Verfahren gemäß Anspruch 10, wobei das Enzym mindestens eines ist, das aus der aus Chitinase und Cellulase bestehenden Gruppe ausgewählt ist.

12. Verfahren gemäß Anspruch 1, wobei das SCF-CO₂-Extraktionsverfahren umfaßt:
Einbringen der sporoderm-aufgebrochenen *Ganoderma*-Sporen in einen Druckbehälter;
Kontaktieren des SCF-CO₂ mit den *Ganoderma*-Sporen im Druckbehälter; und
Druckentlastung des Druckbehälters zum Sammeln der öligen Substanzen aus den sporoderm-aufgebrochenen *Ganoderma*-Sporen.

13. Verfahren gemäß Anspruch 12, wobei der Druckbehälter bei einem Druck zwischen 5 M Psia (Pa) bis 60 M Pa gehalten wird.

14. Verfahren gemäß Anspruch 12, wobei der Druckbehälter bei einer Temperatur von 32°C bis 85°C gehalten wird.

15. Verfahren gemäß Anspruch 12, wobei der Druckbehälter bei einer Durchstromvolumenrate von 5 kg/h bis 80 kg/h gehalten wird.

16. Verfahren gemäß Anspruch 12, wobei die Extraktionsdauer 30 Minuten bis 6 Stunden beträgt.

17. Verfahren gemäß Anspruch 12, wobei die sporoderm-aufgebrochenen *Ganoderma*-Sporen vor dem Einbringen in den Druckbehälter mit einem Träger vermischt werden.

18. Verfahren gemäß Anspruch 17, wobei der Träger 85 bis 100 %-iges Ethanol (Vol/Vol) oder Wasser ist.

19. Verfahren gemäß Anspruch 17, wobei der Träger und die *Ganoderma*-Sporen bei einem Gewichtsverhältnis von 2 % bis 200 % vorliegen.

20. Verfahren gemäß Anspruch 17, ferner umfassend einen Schritt des Trennens der öligen Substanzen von dem Träger durch Zentrifugation.

21. Antitumormittel, umfassend die öligen Substanzen aus *Ga noderma*-Sporen, die gemäß Anspruch 1 erhältlich sind.

22. Anti-HIV-Mittel, umfassend die öligen Substanzen aus *Ganoderma*-Sporen, die gemäß Anspruch 1 erhältlich sind.

23. Anti-HBV-Mittel, umfassend die öligen Substanzen aus *Ga noderma*-Sporen, die gemäß Anspruch 1 erhältlich sind

24. Immunmodulierendes Mittel, umfassend die öligen Substanzen aus *Ganoderma*-Sporen, die gemäß Anspruch 1 erhältlich sind.

25. Verwendung einer wirksamen Menge der öligen Substanzen aus *Ganoderma*-Sporen, die gemäß Anspruch 1 erhältlich sind, zur Herstellung eines Medikaments zum Behandeln eines Patienten mit Tumoren.

26. Verwendung einer wirksamen Menge der öligen Substanzen aus *Ganoderma*-Sporen, die gemäß Anspruch 1 erhältlich sind, zur Herstellung eines Medikaments zum Behandeln eines Patienten mit HIV-Infektion.

27. Verwendung einer wirksamen Menge der öligen Substanzen aus *Ganoderma*-Sporen, die gemäß Anspruch 1 erhältlich sind, zur Herstellung eines Medikaments zum Behandeln eines Patienten mit HBV-Infektion.

28. Verwendung einer wirksamen Menge der öligen Substanzen aus *Ganoderma*-Sporen, die gemäß Anspruch 1 erhältlich sind, zur Herstellung eines Medikaments zum Behandeln eines Patienten mit immunologischen Erkrankungen.

## Revendications

1. Procédé d'extraction des substances oléagineuses des spores de *Ganoderma lucidum* comprenant :
activer par germination lesdites spores de *Ganoderma* ;
casser lesdites spores de *Ganoderma* activées par germination par un moyen mécanique pour obtenir des spores au sporoderme cassé ; et
extraire lesdites substances oléagineuses desdites spores au sporoderme cassé en utilisant un procédé d'extraction au dioxyde de carbone à l'état de fluide supercritique (FSC-CO₂).

2. Procédé selon la revendication 1, dans lequel, avant que lesdites spores de *Ganoderma* ne soient cassées par ledit moyen mécanique, lesdites spores de *Ganoderma* sont activées par germination en trempant lesdites spores de *Ganoderma* dans une solution nutritive qui est appropriée pour l'induction de la germination ; et en plaçant lesdites spores de *Ganoderma* dont la germination a été induite dans une boîte de culture ventilée pour activation jusqu'à ce que les parois cellulaires desdites spores de *Ganoderma* soient ramollies.

3. Procédé selon la revendication 2, dans lequel ladite solution nutritive est au moins une solution choisie dans le groupe consistant en une solution immergée de sporophore de *Ganoderma,* une solution de biotine, de l'eau et une solution immergée de mycélium de *Ganoderma*.

4. Procédé selon la revendication 2, dans lequel lesdites spores de *Ganoderma* sont trempées dans ladite solution nutritive entre 10 minutes et 10 heures.

5. Procédé selon la revendication 2, dans lequel lesdites spores de *Ganoderma* sont trempées dans ladite solution nutritive entre 16 et 43°C.

6. Procédé selon la revendication 2, dans lequel ladite boîte de culture ventilée est à une humidité relative entre 60% et 98%.

7. Procédé selon la revendication 2, dans lequel ladite boîte de culture ventilée est à une température entre 16 et 48°C.

8. Procédé selon la revendication 2, dans lequel lesdites spores de *Ganoderma* trempées sont placées dans ladite boîte de culture ventilée entre 10 minutes et 24 heures.

9. Procédé selon la revendication 1, dans lequel ledit moyen mécanique est au moins un moyen choisi dans le groupe consistant en la micronisation, le courant d'air à ultra haute vitesse, la coupe aux ciseaux/le broyage, et le micro-courant à ultra haute pression.

10. Procédé selon la revendication 1, comprenant en outre une étape de digestion desdites spores de *Ganoderma* avec au moins une enzyme avant d'appliquer ledit moyen mécanique.

11. Procédé selon la revendication 10, dans lequel ladite enzyme est au moins une enzyme choisie dans le groupe consistant en la chitinase et la cellulase.

12. Procédé selon la revendication 1, dans lequel ledit procédé d'extraction FSC-CO₂ comprend :
placer lesdites spores de *Ganoderma* au sporoderme cassé dans un récipient sous pression ;
mettre le FSC-CO₂ au contact desdites spores de *Ganoderma* dans ledit récipient sous pression ; et
dépressuriser ledit récipient sous pression pour collecter lesdites substances oléagineuses desdites spores de *Ganoderma* au sporoderme cassé.

13. Procédé selon la revendication 12, dans lequel ledit récipient sous pression est maintenu à une pression entre 5 M Psia (Pa) à 60 M Pa.

14. Procédé selon la revendication 12, dans lequel ledit récipient sous pression est maintenu à une température de 32°C à 85°C.

15. Procédé selon la revendication 12, dans lequel ledit récipient sous pression est maintenu à un débit en volume de 5 kg/h à 80 kg/h.

16. Procédé selon la revendication 12, dans lequel ledit temps d'extraction est entre 30 minutes et 6 heures.

17. Procédé selon la revendication 12, dans lequel lesdites spores de *Ganoderma* au sporoderme cassé sont mélangées avec un véhicule avant d'être placées dans ledit récipient sous pression.

18. Procédé selon la revendication 17, dans lequel ledit véhicule est 85% à 100% de l'éthanol (en volume) ou de l'eau.

19. Procédé selon la revendication 17, dans lequel ledit véhicule et lesdites spores de *Ganoderma* sont dans une proportion en poids de 2% à 200%.

20. Procédé selon la revendication 17, comprenant en outre une étape de :
séparer lesdites substances oléagineuses dudit véhicule par centrifugation.

21. Agent antitumoral comprenant lesdites substances oléagineuses des spores de *Ganoderma* susceptibles d'être obtenues selon la revendication 1.

22. Agent anti-VIH comprenant lesdites substances oléagineuses des spores de *Ganoderma* susceptibles d'être obtenues selon la revendication 1.

23. Agent anti-VHB comprenant lesdites substances oléagineuses des spores de *Ganoderma* susceptibles d'être obtenues selon la revendication 1.

24. Agent de modulation immun comprenant lesdites substances oléagineuses des spores de *Ganoderma* susceptibles d'être obtenues selon la revendication 1.

25. Utilisation d'une quantité efficace desdites substances oléagineuses des spores de *Ganoderma* susceptibles d'être obtenues selon la revendication 1 pour la préparation d'un médicament destiné à traiter un patient souffrant de tumeurs.

26. Utilisation d'une quantité efficace desdites substances oléagineuses des spores de *Ganoderma* susceptibles d'être obtenues selon la revendication 1 pour la préparation d'un médicament destiné à traiter un patient souffrant d'une infection au VIH.

27. Utilisation d'une quantité efficace desdites substances oléagineuses des spores de *Ganoderma* susceptibles d'être obtenues selon la revendication 1 pour la préparation d'un médicament destiné à traiter un patient souffrant d'une infection au VHB.

28. Utilisation d'une quantité efficace desdites substances oléagineuses des spores de *Ganoderma* susceptibles d'être obtenues selon la revendication 1 pour la préparation d'un médicament destiné à traiter un patient souffrant de troubles immunologiques.
